# EUROPEAN PATENT SPECIFICATION

(11) **EP 3 055 646 B1**
(45) Date of publication and mention of the grant of the patent: **16.12.2020**
(21) Application number: 14772162.5
(22) Date of filing: 24.09.2014
(51) Int. Cl.: G01B 11/16, A61B 5/06, A61B 34/20

(54) **DEVICE TRACKING USING LONGITUDINAL ENCODING**
GERÄTE-TRACKING MIT LÄNGSCODIERUNG
SUIVI DE DISPOSITIF UTILISANT UN CODAGE LONGITUDINAL

(30) Priority: 02.10.2013 EP 13187014
(43) Date of publication of application: 17.08.2016
(73) Proprietor: Koninklijke Philips N.V., 5656 AG Eindhoven (NL)
(72) Inventor: DENISSEN, Sander Hans, NL-5656 AE Eindhoven (NL); VERSTEGE, Marco, NL-5656 AE Eindhoven (NL); DIJKKAMP, Dirk, NL-5656 AE Eindhoven (NL); CNOOPS, Marcellinus, Petrus, Maria, NL-5656 AE Eindhoven (NL)
(74) Representative: Philips Intellectual Property & Standards
(86) International application number: PCT/EP2014/070384
(87) International publication number: WO 2015/049142

(56) References cited:
- WO-A1-2011/141830
- WO-A1-2012/029013
- WO-A2-2012/101584
- US-A1- 2003 135 102
- US-A1- 2005 182 319
- US-A1- 2012 289 843
- US-A1- 2013 188 855
- US-B1- 6 471 710

## Description

### FIELD OF THE INVENTION

The present invention relates to the field of shape sensing, especially three-dimensional (3D) optical shape sensing.

### BACKGROUND OF THE INVENTION

By using optical shape sensing (OSS), the 3D shape of an elongated object, e.g. a steerable medical device, can be reconstructed by integration of an optical fiber with optical shape sensing elements in such a device. This is possible by optical interrogating the optical fiber e.g. with optical shape sensing elements by means of Fiber Bragg Gratings or Rayleigh based elements. A real time visualization of the reconstructed 3D shape has a number of applications, e.g. medical applications, since it allows important navigational guidance for elongated interventional medical devices. Such devices can be used for example within medical applications in the form of diagnostic and navigation devices, e.g. catheters, guide wires, endoscopes, stylets or needles, and treatment devices, e.g. ablation devices.

However, still OSS devices suffer from a number of problems:
1) OSS sensors are expensive, and thus often only one OSS system and one OSS enabled medical device is available for a physician.
2) There is a limited variation of OSS-catheters and some situations require a device with a particular shape, for which device no OSS enabled version is available.
3) When using two OSS-devices, one inside the other, the proximal tethers of OSS devices may twist up when they are rotated around each other. This twisting can make it difficult for the user to handle both devices, and the shape reconstruction could be less accurate and stable due to small bend radius and high strain.

US 2013/188855 A1 discloses a system and a method for tracking a functional part of an instrument during an interventional procedure. The system comprises at least one instrument, a system for acquiring anatomical images, a tether connected to the imaging system at a fixed end and connected to the instrument at a distal end, the tether comprising at least one longitudinal optical fiber with a plurality of optical shape sensors. An optical console interrogates the sensors and detects reflected light. A processor calculates local curvature at each sensor location to determine the three-dimensional shape of the tether and determines the location and orientation of the instrument relative to the images using the local curvature of the tether and the location of the fixed end of the tether.

US 6 471 710 B1 discloses a probe position sensing system for accurately determining the spatial location in a coordinate system of a distal end of the probe assembly. The probe assembly includes an articulated arm having a pair of sections interconnected by a flexible joint and at least one element. The element extends through the joint and is positioned to be subjected to a degree of flexure due to the relative displacement of the sections. A flexure of the element induces a change in a physical property associated with the element. An instrument monitors the physical property and derives an angle between adjacent sections from variations of the physical property.

WO 2011/141830 A1 discloses an optical sensing system employing a flexible optical fiber and an optical fiber controller. The optical fiber includes a deformation optic sensor array having a proximal end point and a distal end point, and may be adjoined to a medical device for generating encoded optical signal indicative of a change in a shape of the optical fiber responsive to movement of the medical device within a defined space. The optical fiber controller utilizes the encoded optical signal for reconstructing a portion or an entirety of a shape of the optical fiber between the proximal end point and the distal end point. To this end, the optical fiber controller segments the optical fiber into an anchor fiber segment and an active fiber segment relative to an anchor point having a fixed sampling location within the defined space as designated by the optical fiber controller.

WO 2012/029013 A1 discloses an optical guide wire system employing an optical guide wire, an optical guide wire controller, a guide interface and an optical connector. The optical guide wire includes one or more guide wire fiber cores for generating an encoded optical signal indicative of a shape of the optical guide wire. The optical connector facilitates connection, disconnection and reconnection of the optical guide wire to the guide wire interface that enables backloading the catheter on the optical guide wire.

WO 2012/101584 A2 discloses a shape sensing device including an interventional instrument having regions of articulation to be configured to change shape during an interventional procedure. An optical fiber is disposed on or about the areas of articulation in a pattern to provide an optical signal indicating an instantaneous change or current position or orientation of the instrument.

US 2003/135102 A1 discloses a system and method for intravascular treatment planning and therapy on a patient. The system includes a structure for holding a treatment source, such as a radiation source, and also moving the treatment source through a patient's vascular system using a positioning device which includes a component to establish the location relative to a particular reference position. An imaging device is also disposed in association with a vascular treatment area and a treatment source to register the resulting image information, enabling formation of three-dimensional image in real-time for use by a clinician.

### SUMMARY OF THE INVENTION

It would be advantageous to provide a method and a system capable of real-time tracking 3D shape of a plurality of different longitudinal devices, e.g. interventional medical catheters, at a low cost, and without the need for a separate scanning device.

In a first aspect, the invention provides an optical shape sensing system comprising
- a guide wire comprising an optical fiber with optical shape sensing properties along at least a part of its longitudinal extension,
- an associated longitudinal device arranged to allow a user to attach the guide wire thereto, so as to ensure that the optical fiber will follow a three-dimensional shape of at least a part of the associated longitudinal device, e.g. a non-OSS medical catheter,
- a longitudinal offset encoding system arranged to establish a recognizable longitudinal offset between the associated longitudinal device (C) and the optical fiber (OF), and
- an optical console system O_C, P) arranged for:
   - recognizing said recognizable longitudinal offset between the associated longitudinal device (C) and the optical fiber (OF), and registering a longitudinal offset of the associated longitudinal device (C) in relation to a point along the optical fiber (OF) accordingly,
   - optically interrogating the optical fiber (OF),
   - reconstructing a three-dimensional shape of at least a part of the optical fiber (OF) in response to a result of said optical interrogation of the optical fiber (OF), and for
   - generating an output (I) indicative of a three-dimensional shape of at least a part of the associated longitudinal device (C) in response to said reconstructed three-dimensional shape of at least part of the optical fiber (OF), and in response to said registered longitudinal offset of the associated longitudinal device (C) in relation to a point along the optical fiber (OF).

By 'associated longitudinal device' is understood a device, e.g. a medical catheter or the like, that can either be non-tracked, i.e. without any shape sensing capability, however if preferred the associated longitudinal device may be a tracked device, e.g. another OSS device. The guide wire with an optical fiber with OSS properties represents a track device, or reference device. The optical fiber is preferably positioned within the guide wire, and the guide wire is then attached to follow the non-tracked associated longitudinal device, e.g. by insertion into a lumen of the non-tracked associated longitudinal device.

It is to be understood that the guide wire comprising the OSS fiber can itself form part of an instrument having an additional function itself, other than merely being able to OSS track an associated longitudinal device. E.g. the guide wire may form part of such as: a navigational catheter, a stent/balloon/contrast injection catheter, or a sheath. Still for such instrument, it can be formed so as to allow the instrument to slide inside or along a side of an associated (non-tracked or tracked) longitudinal device.

The system is advantageous, since with only one OSS system, it is possible to track 3D shape of many different commercially available associated longitudinal devices, e.g. interventional medical catheters without OSS properties. However, it is to be understood that the system can be used in connection with OSS systems where a plurality of devices are tracked. Thus, for medical applications, a number of different thicknesses and lengths of catheters can be used, requiring only one OSS system, even though a system in general may be able to track multiple devices. Still, the advantages of an OSS device remain: e.g. 3D reconstruction of the device allowing multiple viewing angles at the same time (e.g. virtual bi-plane as used in endovascular procedures, or other types of visualization as used in other procedures). Further, it allows real-time device visualization without the use of X-ray or any other imaging modality. Compared to the use of two OSS devices, the aforementioned problem of tangling or twisting is eliminated.

In an example of a practical operation of the system, e.g. for a medical interventional procedure, a user selects an associated longitudinal device, e.g. a catheter among a plurality of possible different catheters, and inserts the guide wire into a lumen of the selected catheter before the medical procedure is performed. By means of the longitudinal offset encoding system, the optical console can automatically detect a longitudinal position on the optical fiber in the guide wire where the catheter is positioned. Thus, via OSS optical interrogation of the optical fiber the 3D shape of the optical fiber can be reconstructed, and with knowledge of the registered longitudinal offset, it is possible to determine which part of the optical fiber that corresponds to the catheter, and thus the catheter 3D shape can finally be determined.

With such system, it is possible to track associated longitudinal device, e.g. medical catheter, which can be manufactured in low cost and thus can be manufactured as disposable products, whereas the guide wire with OSS properties, i.e. the rather expensive component, can be re-used. This allows OSS properties to be used in more applications, e.g. medical applications where a selection of a large number of different longitudinal devices should be available for selection.

In case of a longitudinal device, e.g. a catheter, which has a length equal to or shorter than the available guide wire with optical fiber, the entire length of the longitudinal device can have its shape 3D reconstructed. However, if the longitudinal device is longer than the guide wire with optical fiber, only a part of the length of the longitudinal device, the proximal part, can be tracked. In such cases, it may be possible to estimate a position of the distal port based on the length of the longitudinal device. It may then be possible to estimate an area where the distal part of the longitudinal device, especially its tip, may be present, based on the known shape of the distal portion of the optical fiber. Even though it is not possible to precisely track the tip of the longitudinal device in such cases, it is possible to visually indicate the estimated area to a user.

The registration of the longitudinal offset between of the associated longitudinal device in relation to the optical fiber can be expressed as determining relative longitudinal offset of the associated longitudinal device to the optical fiber. One specific way to do this is by means of a longitudinal offset encoding system comprising a hub to connect to a proximal part of the associated longitudinal device. This hub should have a lumen with a detectable curvature, such that an algorithm in the optical console system can derive from the optical fiber where this hub is in space, i.e. using optical interrogation. The part of the optical fiber that is distal to the hub is known to be inside the associated longitudinal device. Thus, an operating procedure may comprise mounting the guide wire with the optical fiber inside in a hub serving to provide a known curvature of the optical fiber at a known position. This allows an automated way for the optical console system to identify a reference position, e.g. if the guide wire is attached to the associated longitudinal device, such that the proximal end of the associated longitudinal device is positioned at a longitudinal position of the optical fiber immediately after the hub. Especially, the registering may further comprise determining a coordinate system, i.e. determining a data representation allowing a unique identification of a three-dimensional position, thus determining a unique position of a point of the optical fiber in relation to a point of the associated longitudinal device.

There are generally several other ways to implement the longitudinal offset encoding system. Especially, the longitudinal offset encoding system may comprise at least one of: 1) a device arranged to induce strain at the optical fiber at a known position (e.g. as explained above by mounting a hub with known curvature), 2) a system comprising a temperature encoder arranged for positioning at a tip or entry point of the associated longitudinal device (by using the relative temperature-induced strain of a cold bolus or element integrated into the device), 3) a system arranged to generate a temperature between two points of the optical fiber (by using the relative temperature-induced strain in and outside the body), 4) a system arranged to generate strain at two points of the optical fiber (by using a single core optical fiber inside the longitudinal device), and 5) a system comprising an electronic device (e.g. comprising a mechanical potentiometer or an optical sensor, e.g. gradient sensor, discrete sensor).The mentioned longitudinal offset encoding system embodiments are to be understood as a non-exhaustive list.

It is to be understood that the OSS properties of the optical fiber can be obtained in various ways, as known by the skilled persons. E.g. the optical interrogation may make use of Rayleigh scattering, or make use of Fiber Bragg Gratings written into the fiber. The method for optical interrogation of the optical shape sensing properties may be performed in several ways, such as also known by the skilled person. In some embodiments, the optical fiber only has OSS properties in a part of its length. Especially, the optical fiber only has shape sensing properties along a distal part of its longitudinal extension. Thus, a low cost optical fiber can be used, which only has optical shape sensing properties in a limited part of its length, where it is necessary in a given application. Especially, only a distal portion of the optical fiber length may be important to be shape sensed, e.g. to identify a tip position of the associated longitudinal device, or at least to precisely identify a distal position of the optical fiber which may be used for estimating the tip position of the associated longitudinal device.

In the following, a number of embodiments or additional features will be defined.

The longitudinal offset encoding system may comprise a hub arranged for providing a predetermined curvature of the optical fiber at a selected longitudinal position of the optical fiber. Especially, the OSS system may be arranged to recognize said predetermined curvature of the optical fiber by means of optical interrogation, and to detect a point along the optical fiber accordingly, wherein the optical console system is further arranged to register a longitudinal offset of the associated longitudinal device in relation to said detected point along the optical fiber. The hub may be arranged to allow the optical console system to recognize a longitudinal position of a proximal end of the associated longitudinal device on the optical fiber.

The associated longitudinal device may comprise a lumen arranged for insertion of the guide wire (GW). This provides an easy way for the user to attach the guide wire and thus the optical fiber to the associated longitudinal device to ensure that the optical fiber will follow a three-dimensional shape of at least a part of the associated longitudinal device. Such a lumen is present in some existing medical devices, such as catheters (e.g. flushing lumen, guide wire lumen). However, the associated longitudinal device may be manufactured with a specific lumen arranged for accommodating a guide wire of a given dimension. It is to be understood that the guide wire may be attached to the longitudinal device in various other ways, e.g. the guide wire may be attached to an outside part of the longitudinal device. Preferably, the attaching procedure is easy to perform in a short time, thus allowing a user to attach and detach the guide wire to a longitudinal device without any shape sensing properties. E.g. a short attachment time will allow a physician to decide which one of a plurality of medical devices to use in a medical examination at a late point in time before the examination.

The system may be arranged for entering of at least one property of the associated longitudinal device comprising at least one of: a length, a thickness, a color, and a type, and wherein the optical shape sensing system is arranged for utilizing this at least one property of the associated longitudinal device for visualizing the three-dimensional shape of the associated longitudinal device to a user. This information may be obtained directly from the device, by reading out a digital storage medium on the device or from a database where the device has a unique ID, which can be obtained by scanning a bar code, a built-in RFID tag, or other identification marker of the associated longitudinal device, thereby entering one or more properties of the specific longitudinal device into a control software of an OSS system. The one or more properties may alternatively or additionally be manually entered by a user, e.g. entering a length of the longitudinal device and/or a name, a type, or an identification number of the longitudinal device. The properties may be reflected in a visualization of the 3D shape of the longitudinal device to a user.

The guide wire with the OSS properties may have a length being smaller than a length of the associated longitudinal device. In such case, it is not possible to precisely track a distal part of the associated longitudinal device. Therefore, the system may in such cases be arranged to estimate a measure of three-dimensional shape of a distal end of the associated longitudinal device in response to a reconstructed shape of the optical fiber, and in response to a length of the associated longitudinal device. Especially, in response to a reconstructed end point of the optical fiber, and in response to a length of the associated longitudinal device. The end point, and e.g. end portion, of the optical fiber may be used for the estimation, since e.g. with a known maximum possible curvature of the longitudinal device, a special angle can be calculated in which the remaining length of the longitudinal device will probably be. Especially, the system may be arranged to generate an image with a graphical indication of said estimated measure of 3D shape of the distal end of the associated longitudinal device. E.g. the precisely tracked 3D shape may be indicated visually to a user in one way, whereas the estimated part may be indicated in another way, thus informing the user that the precise position of the distal part of the longitudinal device is unknown. More specifically, said graphical indication may comprise at least one of: a line around an area where the distal part of the associated longitudinal device will most likely be, an intensity or color code indicating where the distal part of the associated longitudinal device will most likely be, and sketches of a plurality of possible paths the distal part of the associated longitudinal device may follow.

Especially, the system may be arranged to estimate a measure of three-dimensional shape of a distal end of the associated longitudinal device in response to a model of a default shape of the associated longitudinal device. E.g. the distal part of the longitudinal device may be rather stiff, thus there is no need to optically track the full length of such longitudinal device, since the position and shape of such distal part can be estimated with a high accuracy based on 3D tracking of the proximal part of the longitudinal device.

The optical fiber may only have shape sensing properties long a distal part of its longitudinal extension. Hereby, the optical fiber can be manufactured with lower costs, and often only the distal part of the associated longitudinal device may be interesting to OSS track.

The optical fiber is preferably protected by a tubing forming part of the guide wire, e.g. in the form of a thin medical device guide wire. Thereby, the optical fiber is less fragile, and thus it is easier to handle in the manual procedure of attaching and detaching the OSS optical fiber.

The system is suited for as a real-time automated OSS system, and the step of visualizing the shape of the associated longitudinal device as the reconstructed shape of the optical fiber, can be implemented in software.

The system may comprise a plurality of medical catheters that can be selected as the associated longitudinal device. Thus, e.g. a medical procedure, may comprise selecting the associated longitudinal device to be one of a plurality of possible catheters. This can be relevant for a physician who may select between a number of different catheters without shape sensing properties, and then select the relevant one for a specific medical examination and/or treatment.

Especially, the system is a medical system, i.e. a system arranged for medical examination and/or medical treatment.

In a second aspect, the invention provides a method for reconstructing a shape of an associated longitudinal device, the method comprising
- providing a guide wire with an optical fiber with optical shape sensing properties along at least a part of its longitudinal extension,
- attaching the guide wire to at least a part of an associated longitudinal device, so as to ensure that the optical fiber will follow a three-dimensional shape of at least a part of the associated longitudinal device,
- establishing a recognizable longitudinal offset between the associated longitudinal device and the optical fiber, and
- recognizing said recognizable offset between the associated longitudinal device and the optical fiber, and registering a longitudinal offset of the associated longitudinal device in relation to a point along the optical fiber accordingly,
- optically interrogating the optical fiber,
- reconstructing a three-dimensional shape of at least a part of the optical fiber in response to a result of said optical interrogation of the optical fiber, and
- generating an output indicative of a three-dimensional shape of at least a part of the associated longitudinal device in response to said reconstructed three-dimensional shape of at least a part of the optical fiber, and in response to said registered longitudinal offset of the associated longitudinal device in relation to a point along the optical fiber.

In a third aspect, the invention provides a computer executable program code. The computer program comprises code means for causing a computer to carry out the steps:
- to register data indicating a longitudinal offset between an associated longitudinal device and an optical fiber with optical shape sensing properties, wherein the optical fiber forms part of a guide wire,
- to receive data from an optical interrogation of the optical shape sensing properties of the optical fiber,
- to reconstruct a three-dimensional shape of at least a part of the optical fiber in response to said data from the optical interrogation, and
- to generate an output indicative of a three-dimensional shape of at least a part of the associated longitudinal device in response to said three-dimensional shape of at least a part of the optical fiber, and in response to said longitudinal offset of the associated longitudinal device in relation to the optical fiber.

Such computer executable program code is thus capable of performing the steps of the method according to the second aspect which can be implemented in software, e.g. as an add-on or modification of existing OSS software. Preferably, the program code allows entering of properties of the longitudinal device, either automatically using an identification code of the longitudinal device, or by manual entering e.g. a length of the longitudinal device etc.

The computer executable program code may especially be present on a non-transitory computer readable storage medium, or it may be loaded into memory of a processor system arranged to execute the program code.

It is appreciated that the same advantages and embodiments of the first aspect apply as well for the second and third aspect. In general the first, second, and third aspects may be combined and coupled in any way possible within the scope of the invention, the scope of the invention being defined in the appended claims. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Fig. 1 illustrates an embodiment where an OSS fiber is shorter than a catheter in which it is used,
Fig. 2 illustrates an embodiment where an OSS fiber is longer than a catheter in which it is used,
Fig. 3 illustrates one way of indicating a zone in which a remaining part of a catheter will most likely be positioned,
Fig. 4 illustrates another way of indicating a zone in which a remaining part of a catheter will most likely be positioned,
Fig. 5 illustrates still another way of indicating a zone in which a remaining part of a catheter will most likely be positioned, using information about a biological environment e.g. blood vessel geometry,
Fig. 6 illustrates a photo of an implementation of a hub for position registration,
Fig. 7 illustrates sketches of three different curvatures which can be used in a hub for position registration,
Fig. 8 illustrates an example of parts of an OSS system, and
Fig. 9 illustrates a diagram of steps of a method embodiment.

### DESCRIPTION OF EMBODIMENTS

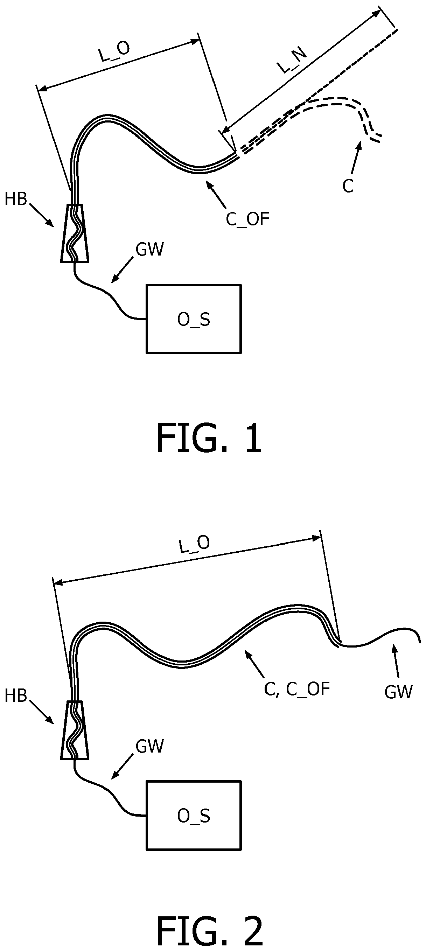

Fig. 1 illustrates an OSS system embodiment where an optical fiber arranged inside a guide wire GW is connected to an optical console system O_S in one end. The distal end of the guide wire GW is positioned inside an associated longitudinal device C in the form of a catheter without shape sensing properties, and having a length of L_O+L_N. A hub HB is mounted to provide a known and trackable curvature of the guide wire GW and thus also the optical fiber therein at a proximal end of the catheter C, hereby allowing a registration of the proximal end of the catheter C to a longitudinal position of the optical fiber inside the guide wire GW. The optical fiber has only OSS properties along its distal length, namely the length L_O, which is only a part of the total length L_O+L_N of the catheter C. Thus, only the proximal part C_OF of the catheter has the optical fiber inside, whereas the distal part of the catheter C has not. Thus, the actual shape of the distal part of the catheter C cannot be tracked. However, since the total length of the catheter C is known, the distal part can be indicated graphically to a user, here shown as a straight dashed line indicating the length L_N of the remaining part of the catheter C, and pointing in a 3D direction determined by the reconstructed end point of the optical fiber. This may be more valuable information for the user compared to simply not showing the remaining part of the catheter. In addition to or alternatively to the line with a length indicating the remaining catheter length L_N, a label can be used to indicate the remaining length, e.g. in mm, e.g. with an arrow pointing to the end point of the shape sensed part C_OF.

It may be possible to provide a better estimate, e.g. if the mechanical properties, e.g. stiffness, of the catheter is known.

Fig. 2 shows the same principal OSS system as in Fig. 1, however here the guide wire GW with an optical fiber inside with optical shape sensing properties extends in the entire part C_OF of the catheter C, and even in a further part. Thus, the total length L_O of the catheter can be shape sensed using the optical fiber in the guide wire GW and the OSS console system O_S. Thus, preferably the 3D shape of the full catheter length is visualized to the user.

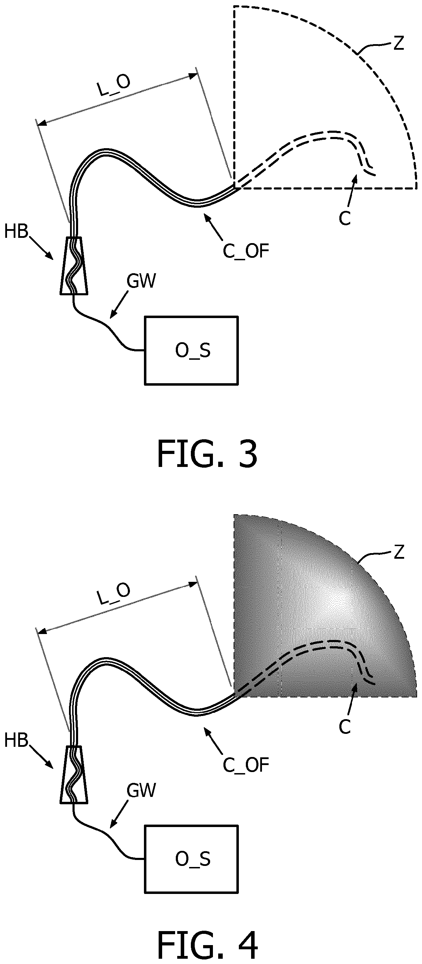

Figs 3 and 4 shows the same system as in Fig. 1, i.e. with a catheter C where a distal part exceeds the length L_O of the catheter which can be 3D shape sensed by an attached guide wire GW with an OSS optical fiber and connected hub HB and optical console system O_S.

Fig. 3 shows indication of an area Z with dashed lines, where the remaining part of the catheter C will most likely be present. The area Z, or angular space, is preferably determined based on the reconstructed end point of the shape sensed part C_OF of the catheter. The tip of the device does not have to be in the quarter circle indicated: not taking a bend radius into account, the tip can be somewhere in a full circle. However, based on the bend radius and expected progress of the device, the range can be limited.

Fig. 4 shows the same area Z, but here indicated with a colored or shaded area, where the color, shading or intensity indicates a probability of where the non-sensed part of the catheter C will most likely be.

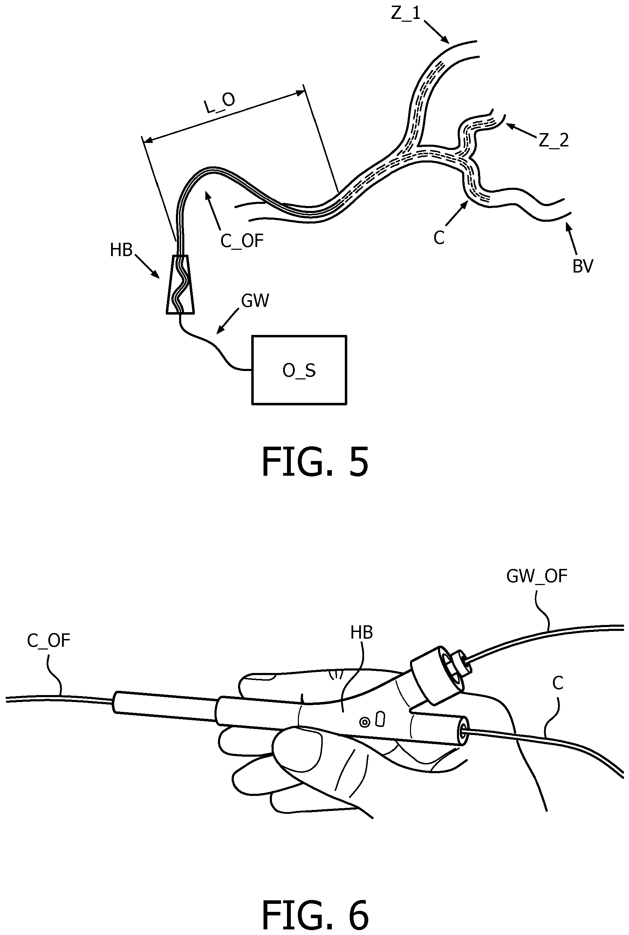

Fig. 5 shows again the same principal system as in Fig. 1, but where the position of the remaining part of the catheter C, i.e. the non-shape sensed part, is indicated as dashed lines C, Z_1, Z_2 following different paths of a lumen segment, e.g. a blood vessel or a broncus segment, BV from the end point of the shape sensed part C_OF of the catheter. Information about the lumen segment can come from modalities such as X-ray (contrast run) or (registered) 3D volumes (CT/MR) or ultrasound. With a known length of the remaining part of the catheter, and with a known plurality of possible paths C, Z_1, Z_2, e.g. here in segments BV, the user can get a more meaningful illustration of where the tip of the catheter may be.

Fig. 6 shows an example of a hub HB having a straight tube section where a longitudinal device, e.g. a catheter C, enters in one end and another tube section with a curved portion, where an optical fiber arranged inside a guide wire GW_OF enters. The guide wire GW_OF enters the catheter C inside the hub HB, and thus the catheter C_OF with the guide wire GW_OF inside, and thereby also the optical fiber inside, is present at the output tube of the hub HB. The combination of curved and straight sections create a unique and identifiable curve shape, thereby enabling registration of a common point between the guide wire GW_OF and the catheter C. In the illustration, the hub HB is not placed at the proximal end of the catheter C, however this could be the case, thus allowing identification of the proximal end of the catheter C as registration point.

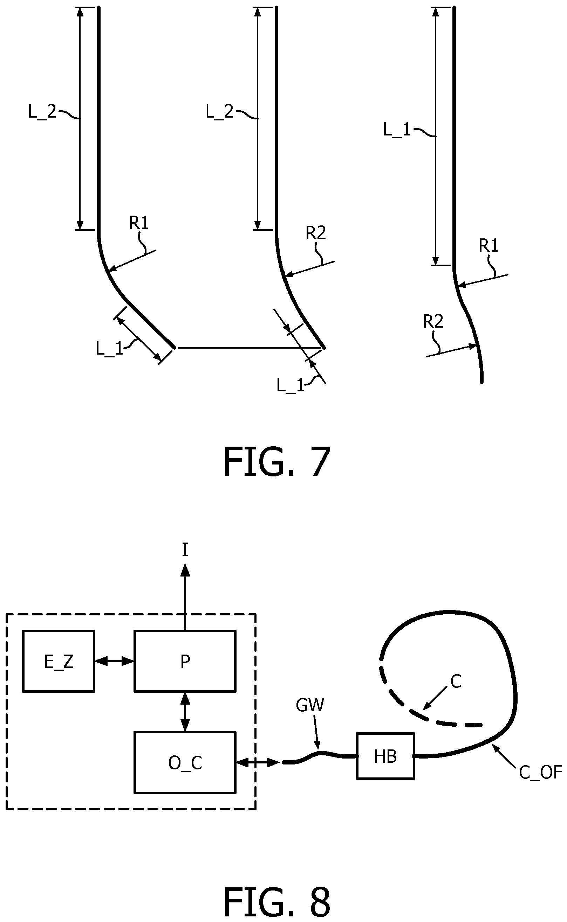

Fig. 7 illustrates three different path examples that can be used for an optical fiber path in a hub. The example to the left has a first rather short straight portion L_1 followed by a curved portion with curvature radius R1, and then followed by a rather long straight section L_2. The example in the middle is similar except for the lengths of L_1, L_2 and the curvature radius R2 which is larger than R1. The example to the right has a combination of two oppositely curved portions with curvature radius R1 and R2, followed by a rather long straight section L_1. The overall lengths of the shown examples may be such as 80-100 mm. The long straight sections L_2, L_2, and L_1, respectively, may have a length of such as 50-70 mm, e.g. around 60 mm.

When an optical fiber with OSS properties follows the unique path, it is possible with the OSS technique to identify the unique and known path, and thereby identify a point which can be used for registration, e.g. the point may be the proximal end of the catheter which could be just after the long straight sections L_2, L_2, L_1, respectively, in the shown path examples.

Fig. 8 shows an OSS system embodiment with an optical fiber with OSS properties placed inside a guide wire GW which is inserted into a lumen of a longitudinal device in the form of a catheter C, and thus the optical fiber will follow the shape of the catheter C. The catheter C is shown to be longer than the guide wire GW and thus also longer than the optical fiber, and thus the distal end part of the catheter, shown with dashed line, is not possible to trace with OSS. Only the proximal end C_OF of the catheter C can be OSS tracked. A hub HB with a known unique curvature is used to identify the proximal end of the catheter C as registration point. The optical fiber is optically inside the guide wire GW is connected to an optical console system O_C arranged for registering said registration point. The optical console O_C is arranged to optically interrogating the optical shape sensing properties of the optical fiber in the guide wire GW, and for reconstructing a 3D shape of at least a part of the optical fiber in response to said optical interrogation, such as known by the skilled person. E.g. using interferometric methods. Thus, the 3D shape of the optical fiber inside the guide wire can be reconstructed, and since it follows the shape of the catheter C, a processor P generates an output image I indicative of 3D shape of the proximal part of the catheter which is identical to the reconstructed 3D shape of the optical fiber. Further, since the distal part of the catheter C cannot be precisely reconstructed, the processor P executes an estimation algorithm E_Z that calculates an estimated area where it is most likely, based at least on knowledge of a total length of the catheter, and on the orientation of the end point of the optical fiber, where the distal part C of the catheter will most likely be positioned. This is graphically included in the output image I, e.g. using a colored or shaded area, thus giving a user, e.g. a physician, an indication of where the distal part of the catheter C may be. Preferably, the image I is created in real-time, thus allowing the OSS system to be used in medical applications to assist a physician in guiding positioning of an interventional catheter during examination and/or treatment when the image I is displayed on a display.

A possible workflow for practical use in a medical application may be:
1. A clinician has a patient on a table and has registered an OSS guide wire available with an OSS fiber inside.
2. The clinician gets a catheter with a suitable length and thickness, and enters the correct length of the catheter, and possibly also other characteristics of the catheter as well, into the software of the OSS system.
3. The clinician connects a hub with described features to the proximal part of the catheter.
4. The clinician enters the OSS guide wire into a lumen of the catheter, and as soon as the OSS enters the catheter, the overlap is displayed, and an indication of the remainder of the catheter can be shown.

In the shown embodiment, it is possible to shape sense only the distal part of the optical fiber, e.g. using a limited Bragg pattern, and use longitudinal encoding for the remaining shape. The Bragg grated optical fiber is rather expensive, so minimizing its length will reduce the total cost of the system. Other technologies such as discrete Bragg or EM tracking can be used for the distal part of the catheter C, and longitudinal encoding for the remaining part.

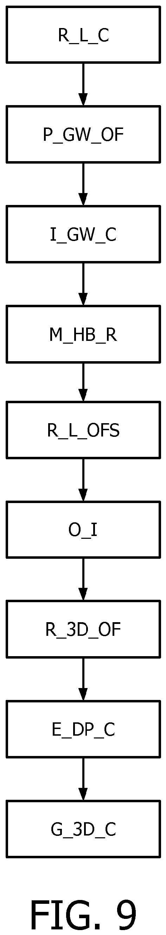

Fig. 9 illustrates steps of a specific method embodiment where the associated longitudinal device is a catheter, e.g. a medical catheter, without shape sensing properties. First step R_L_C is registration of a length of a catheter, and possibly further , e.g. a code or other identification of a specific catheter. This may be manually entered using a user interface of the associated OSS system. Next step P_GW_OF is to provide a guide wire with an optical fiber with OSS properties. Then, the method comprises the step I_GW_C of inserting the guide wire into a lumen in the catheter suited for receiving the guide wire. Especially, the catheter may be longer than the optical fiber, thus the optical fiber only extends in a proximal part of the catheter.

Then, the method comprises the step M_HB_R of mounting a known hub on the guide wire at a position immediately adjacent to the proximal end of the catheter, thereby providing a known curvature of the optical fiber to allow the associated OSS system to register a point on the optical fiber to a proximal end point of the catheter, and thus establishing a recognizable longitudinal offset between the associated longitudinal device and the optical fiber. Further, the method comprises recognizing R_ L_OFS said recognizable offset between the associated longitudinal device and the optical fiber, by means of optical interrogation to identify the curvature of the hub, and thereby identifying the longitudinal offset. This longitudinal offset of the associated longitudinal device in relation to a point along the optical fiber is then registered accordingly.

The step O_I of performing an optical interrogation of the optical fiber and the step R_3D_OF of 3D shape reconstruction of at least a part of the optical fiber in response to said data from the optical interrogation can be performed continuously in real-time by, thus allowing a real-time update of the 3D shape on a display. Next, the step E_DP_C of estimating a position and shape of a distal part of the catheter is performed. I.e. the part of the catheter exceeding the length of the optical fiber, and thus without precise OSS tracking possibility. Finally, the step G_3D_C of generating an output indicative of a 3D shape of at least a part of the associated longitudinal device in response to said 3D shape of at least a part of the optical fiber, and in response to said longitudinal offset of the associated longitudinal device in relation to the optical fiber. This output may be in the form of a 3D image of the catheter. This image preferably shows the precisely tracked shape of the catheter with a different graphical property than the estimated part of the catheter which may be indicated e.g. with a dotted line, or with an indication of a spherical angle, where it is estimated that the distal part of the catheter is present.

Steps E_DP_C and G_3D_C are preferably also performed in real-time and presented to a user, e.g. a physician, so as to enable a real-time feedback on the position of the catheter during use.

To sum up, the invention provides a method for reconstructing 3D shape of a longitudinal device using an optical fiber with optical shape sensing (OSS) properties, e.g. Bragg gratings. By attaching the optical fiber to the longitudinal device, such that the optical fiber follows its 3D shape upon bending, known OSS techniques can be applied to reconstruct 3D shape of the optical fiber, and thus also the longitudinal device, e.g. a medical catheter. E.g. the optical fiber, e.g. placed in a guide wire, can be inserted in a lumen of the longitudinal device. Hereby, one OSS system can be used for 3D tracking a plurality of non-shape sensed catheters or other longitudinal devices. In case the longitudinal device is longer than the optical fiber, the position and shape of the remaining part of the longitudinal device may be estimated and visualized to a user, e.g. based on a known length of the longitudinal device, and based on an orientation of an end point of the optical fiber, e.g. using knowledge about the stiffness or other properties of the longitudinal device.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An optical shape sensing system comprising:
- a guide wire (GW) comprising an optical fiber (OF) with optical shape sensing properties along at least a part of its longitudinal extension,
- an associated longitudinal device (C) arranged to allow a user to attach the guide wire thereto, so as to ensure that the optical fiber (OF) will follow a three-dimensional shape of at least a part of the associated longitudinal device (C),
- a longitudinal offset encoding system (HB) arranged to establish a recognizable longitudinal offset between the associated longitudinal device (C) and the optical fiber (OF), and
- an optical console system O_C, P) arranged for:
- recognizing said recognizable longitudinal offset between the associated longitudinal device (C) and the optical fiber (OF), and registering a longitudinal offset of the associated longitudinal device (C) in relation to a point along the optical fiber (OF) accordingly,
- optically interrogating the optical fiber (OF),
- reconstructing a three-dimensional shape of at least a part of the optical fiber (OF) in response to a result of said optical interrogation of the optical fiber (OF), and for
- generating an output (I) indicative of a three-dimensional shape of at least a part of the associated longitudinal device (C) in response to said reconstructed three-dimensional shape of at least part of the optical fiber (OF), and in response to said registered longitudinal offset of the associated longitudinal device (C) in relation to a point along the optical fiber (OF).

2. Optical shape sensing system according to claim 1, wherein the associated longitudinal device (C) comprises a lumen arranged for insertion of the guide wire (GW), so as to ensure that the optical fiber (OF) will follow a three-dimensional shape of at least a part (C_OF) of the associated longitudinal device (C).

3. Optical shape sensing system according to claim 1, arranged for entering of at least one property of the associated longitudinal device comprising at least one of: a length, a thickness, a color, and a type, and wherein the optical shape sensing system is arranged for utilizing this at least one property of the associated longitudinal device for visualizing the three-dimensional shape of the associated longitudinal device to a user.

4. Optical shape sensing system according to claim 1, wherein the guide wire (GW) has a length being smaller than a length of the associated longitudinal device (C).

5. Optical shape sensing system according to claim 4, wherein the system is arranged to estimate a measure of three-dimensional shape of a distal end of the associated longitudinal device in response to a reconstructed shape of the optical fiber (OF), and in response to a length of the associated longitudinal device (C).

6. Optical shape sensing system according to claim 5, wherein the system is arranged to generate an image with a graphical indication of said estimated measure of three-dimensional shape of the distal end of the associated longitudinal device (C).

7. Optical shape sensing system according to claim 6, wherein said graphical indication comprises at least one of: a line around an area where the distal part of the associated longitudinal device (C) will most likely be, an intensity or color code indicating where the distal part of the associated longitudinal device (C) will most likely be, and sketches of a plurality of possible paths the distal part of the associated longitudinal device (C) may follow.

8. Optical shape sensing system according to claim 1, wherein the system is arranged to estimate a measure of three-dimensional shape of a distal end of the associated longitudinal device (C) in response to a model of a default shape of the associated longitudinal device (C).

9. Optical shape sensing system according to claim 1, wherein the longitudinal offset encoding system comprises at least one of: 1) a device arranged to induce strain at the optical fiber (OF) at a known position, 2) a system comprising a temperature encoder arranged for positioning at a tip or entry point of the associated longitudinal device (C), 3) a system arranged to generate a temperature between two points of the optical fiber (OF), 4) a system arranged to generate strain at two points of the optical fiber (OF), and 5) a system comprising an electronic device.

10. Optical shape sensing system according to claim 1, wherein the longitudinal offset encoding system comprises a hub (HB) arranged for providing a predetermined curvature of the optical fiber (OF) at a selected longitudinal position of the optical fiber (OF).

11. Optical shape sensing system according to claim 10, wherein the optical console system O_C, P) is arranged to recognize said predetermined curvature of the optical fiber (OF) by means of optical interrogation, and to detect a point along the optical fiber (OF) accordingly, wherein the optical console system O_C, P) is further arranged to register a longitudinal offset of the associated longitudinal device (C) in relation to said detected point along the optical fiber (OF).

12. Optical shape sensing system according to claim 10, wherein the hub (HB) is arranged to allow the optical console system O_C, P) to recognize a longitudinal position of a proximal end of the associated longitudinal device (C) on the optical fiber (OF).

13. Optical shape sensing system according to claim 1, further comprising a plurality of medical catheters that can be selected as the associated longitudinal device (C).

14. A method for reconstructing a shape of an associated longitudinal device, the method comprising:
- providing (P_GW OF) a guide wire (GW) with an optical fiber (OF) with optical shape sensing properties along at least a part of its longitudinal extension,
- attaching (I_GW_C) the guide wire (GW) to at least a part of an associated longitudinal device (C), so as to ensure that the optical fiber (OF) will follow a three-dimensional shape of at least a part of the associated longitudinal device (C),
- establishing (M_HB_R) a recognizable longitudinal offset between the associated longitudinal device (C) and the optical fiber (OF), and
- recognizing (R_L_OFS) said recognizable offset between the associated longitudinal device (C) and the optical fiber (OF), and registering a longitudinal offset of the associated longitudinal device (C) in relation to a point along the optical fiber (OF) accordingly,
- optically interrogating (O_1) the optical fiber (OF),
- reconstructing (R_3D_OF) a three-dimensional shape of at least a part of the optical fiber (OF) in response to a result of said optical interrogation of the optical fiber (OF, and
- generating (G_3D_C) an output (I) indicative of a three-dimensional shape of at least a part of the associated longitudinal device (C) in response to said reconstructed three-dimensional shape of at least a part of the optical fiber (OF), and in response to said registered longitudinal offset of the associated longitudinal device (C) in relation to a point along the optical fiber (OF).

15. A computer program comprising code means for causing a computer to carry out the steps:
- to register data (M_HB_R) indicating a longitudinal offset between an associated longitudinal device and an optical fiber with optical shape sensing properties, wherein the optical fiber forms part of a guide wire,
- to receive data (O_I) from an optical interrogation of the optical shape sensing properties of the optical fiber,
- to reconstruct (R 3D_OF) a three-dimensional shape of at least a part of the optical fiber in response to said data from the optical interrogation, and
- to generate an output (G_3D_C) indicative of a three-dimensional shape of at least a part of the associated longitudinal device in response to said three-dimensional shape of at least a part of the optical fiber, and in response to said longitudinal offset of the associated longitudinal device in relation to the optical fiber.

## Patentansprüche

1. Optisches Formabtastungssystem, umfassend:
- einen Führungsdraht (GW), der eine optische Faser (OF) mit optischen Formabtastungseigenschaften entlang zumindest eines Teils seines Längsverlaufs umfasst,
- eine zugehörige längsgerichtete Vorrichtung (C), die dafür ausgelegt ist, einem Anwender zu ermöglichen, den Führungsdraht daran anzubringen, um sicherzustellen, dass die optische Faser (OF) einer dreidimensionalen Form von zumindest einem Teil der zugehörigen längsgerichteten Vorrichtung (C) folgt,
- ein Längsrichtungsversatz-Kodiersystem (HB), das dafür ausgelegt ist, einen erkennbaren Längsrichtungsversatz zwischen der zugehörigen längsgerichteten Vorrichtung (C) und der optischen Faser (OF) einzurichten, und
- ein optisches Konsolensystem (O_C, P), das ausgelegt ist zum:
- Erkennen des erkennbaren Längsrichtungsversatzes zwischen der zugehörigen längsgerichteten Vorrichtung (C) und der optischen Faser (OF) und dementsprechend Registrieren eines Längsrichtungsversatzes der zugehörigen längsgerichteten Vorrichtung (C) in Bezug auf einen Punkt entlang der optischen Faser (OF),
- optisch Abfragen der optischen Faser (OF),
- Rekonstruieren einer dreidimensionalen Form von zumindest einem Teil der optischen Faser (OF) als Reaktion auf ein Ergebnis der optischen Abfragung der optischen Faser (OF) und zum
- Erzeugen einer Ausgabe (I), die eine dreidimensionale Form von zumindest einem Teil der zugehörigen längsgerichteten Vorrichtung (C) angibt, als Reaktion auf die rekonstruierte dreidimensionale Form von zumindest dem Teil der optischen Faser (OF) und als Reaktion auf den registrierten Längsrichtungsversatz der zugehörigen längsgerichteten Vorrichtung (C) in Bezug auf einen Punkt entlang der optischen Faser (OF) .

2. Optisches Formabtastungssystem nach Anspruch 1, wobei die zugehörige längsgerichtete Vorrichtung (C) ein Lumen umfasst, das für die Einführung des Führungsdrahts (GW) ausgelegt ist, um sicherzustellen, dass die optische Faser (OF) einer dreidimensionalen Form von zumindest einem Teil (C_OF) der zugehörigen längsgerichteten Vorrichtung (C) folgt.

3. Optisches Formabtastungssystem nach Anspruch 1, das für die Eingabe mindestens einer Eigenschaft der zugehörigen längsgerichteten Vorrichtung geeignet ist, die mindestens eines umfasst von: einer Länge, einer Dicke, einer Farbe und einem Typ, und wobei das optische Formabtastungssystem dafür ausgelegt ist, diese mindestens eine Eigenschaft der zugehörigen längsgerichteten Vorrichtung für die Visualisierung der dreidimensionalen Form der zugehörigen längsgerichteten Vorrichtung für einen Anwender zu nutzen.

4. Optisches Formabtastungssystem nach Anspruch 1, wobei der Führungsdraht (GW) eine Länge aufweist, die kleiner ist als eine Länge der zugehörigen längsgerichteten Vorrichtung (C).

5. Optisches Formabtastungssystem nach Anspruch 4, wobei das System dafür ausgelegt ist, eine Abmessung einer dreidimensionalen Form eines distalen Endes der zugehörigen längsgerichteten Vorrichtung als Reaktion auf eine rekonstruierte Form der optischen Faser (OF) und als Reaktion auf eine Länge der zugehörigen längsgerichteten Vorrichtung (C) zu schätzen.

6. Optisches Formabtastungssystem nach Anspruch 5, wobei das System dafür ausgelegt ist, ein Bild mit einer grafischen Angabe der geschätzten Abmessung der dreidimensionalen Form des distalen Endes der zugehörigen längsgerichteten Vorrichtung (C) zu erzeugen.

7. Optisches Formabtastungssystem nach Anspruch 6, wobei die grafische Angabe mindestens eines umfasst von: einer Linie um einen Bereich, wo sich der distale Teil der zugehörigen längsgerichteten Vorrichtung (C) am wahrscheinlichsten befindet, einer Intensität oder eines Farbcodes, die bzw. der angibt, wo sich der distale Teil der zugehörigen längsgerichteten Vorrichtung (C) am wahrscheinlichsten befindet, und Skizzen einer Vielzahl möglicher Wege, denen der distale Teil der zugehörigen längsgerichteten Vorrichtung (C) folgen kann.

8. Optisches Formabtastungssystem nach Anspruch 1, wobei das System dafür ausgelegt ist, eine Abmessung einer dreidimensionalen Form eines distalen Endes der zugehörigen längsgerichteten Vorrichtung (C) als Reaktion auf ein Modell einer voreingestellten Form der zugehörigen längsgerichteten Vorrichtung (C) zu schätzen.

9. Optisches Formabtastungssystem nach Anspruch 1, wobei das Längsrichtungsversatz-Kodiersystem mindestens eines umfasst von: 1) einer Vorrichtung, die dafür ausgelegt ist, an einer bekannten Position einen Zug an einer optischen Faser (OF) hervorzurufen, 2) ein System, das einen Temperaturkodierer umfasst, der dafür konfiguriert ist, eine Spitze oder einen Eintrittspunkt der zugehörigen längsgerichteten Vorrichtung (C) zu positionieren, 3) ein System, das dafür ausgelegt ist, eine Temperatur zwischen zwei Punkten der optischen Faser (OF) zu erzeugen, 4) ein System, das dafür ausgelegt ist, einen Zug an zwei Punkten der optischen Faser (OF) zu erzeugen, und 5) ein System, das eine elektronische Vorrichtung umfasst.

10. Optisches Formabtastungssystem nach Anspruch 1, wobei das Längsrichtungsversatz-Kodiersystem einen Hub (HB) umfasst, der dafür ausgelegt ist, eine vorgegebene Krümmung der optischen Faser (OF) an einer ausgewählten Position in Längsrichtung der optischen Faser (OF) bereitzustellen.

11. Optisches Formabtastungssystem nach Anspruch 10, wobei das optische Konsolensystem (0_C, P) dafür ausgelegt ist, die vorgegebene Krümmung der optischen Faser (OF) mittels optischer Abfragung zu erkennen und dementsprechend einen Punkt entlang der optischen Faser (OF) zu erfassen, wobei das optische Konsolensystem (O_C, P) ferner dafür ausgelegt ist, einen Längsrichtungsversatz der zugehörigen längsgerichteten Vorrichtung (C) in Bezug auf den erfassten Punkt entlang der optischen Faser (OF) zu registrieren.

12. Optisches Formabtastungssystem nach Anspruch 10, wobei der Hub (HB) dafür ausgelegt ist, dem optischen Konsolensystem (O_C, P) eine Erkennung einer Längsrichtungsposition eines proximalen Endes der zugehörigen längsgerichteten Vorrichtung (C) an der optischen Faser (OF) zu ermöglichen.

13. Optisches Formabtastungssystem nach Anspruch 1, ferner eine Vielzahl von medizinischen Kathetern umfassend, die als die zugehörige längsgerichtete Vorrichtung (C) ausgewählt werden können.

14. Verfahren zum Rekonstruieren einer Form einer zugehörigen längsgerichteten Vorrichtung, wobei das Verfahren umfasst:
- Ausstatten (P_GW_OF) eines Führungsdrahts (GW) mit einer optischen Faser (OF) mit optischen Formabtastungseigenschaften entlang zumindest eines Teils seines Längsrichtungsverlaufs,
- Anbringen (I_GW_C) des Führungsdrahts (GW) an zumindest einem Teil einer zugehörigen längsgerichteten Vorrichtung (C), um sicherzustellen, dass die optische Faser (OF) einer dreidimensionalen Form von zumindest einem Teil der zugehörigen längsgerichteten Vorrichtung (C) folgt,
- Einrichten (M_HB_R) eines erkennbaren Versatzes zwischen der zugehörigen längsgerichteten Vorrichtung (C) und der optischen Faser (OF), und
- Erkennen (R_L_OFS) des erkennbaren Versatzes zwischen der zugehörigen längsgerichteten Vorrichtung (C) und der optischen Faser (OF) und dementsprechend Registrieren eines Längsrichtungsversatzes der zugehörigen längsgerichteten Vorrichtung (C) in Bezug auf einen Punkt entlang der optischen Faser (OF),
- optisches Abfragen (O_I) der optischen Faser (OF) ,
- Rekonstruieren (R_3D_OF) einer dreidimensionalen Form von zumindest einem Teil der optischen Faser (OF) als Reaktion auf ein Ergebnis der optischen Abfragung der optischen Faser (OF), und
- Erzeugen (G_3D_C) einer Ausgabe (I), die eine dreidimensionale Form von zumindest einem Teil der zugehörigen längsgerichteten Vorrichtung (C) angibt, als Reaktion auf die rekonstruierte dreidimensionale Form von zumindest dem Teil der optischen Faser (OF) und als Reaktion auf den registrierten Längsrichtungsversatz der zugehörigen längsgerichteten Vorrichtung (C) in Bezug auf einen Punkt entlang der optischen Faser (OF) .

15. Computerprogramm, eine Code-Einrichtung umfassend, um einen Computer zu veranlassen, die folgenden Schritte auszuführen:
- Registrieren von Daten (M_HB_R) , die einen Längsrichtungsversatz zwischen einer zugehörigen längsgerichteten Vorrichtung und einer optischen Faser mit optischen Formabtastungseigenschaften angeben, wobei die optische Faser einen Teil eines Führungsdrahts bildet,
- Empfangen von Daten (O_I) aus einer optischen Abfragung der optischen Formabtastungseigenschaften der optischen Faser,
- Rekonstruieren (R_3D_OF) einer dreidimensionalen Form von zumindest einem Teil der optischen Faser als Reaktion auf die Daten aus der optischen Abfragung und
- Erzeugen einer Ausgabe (G_3D_C), die eine dreidimensionale Form von zumindest einem Teil der zugehörigen längsgerichteten Vorrichtung angibt, als Reaktion auf die dreidimensionale Form von zumindest dem Teil der optischen Faser und als Reaktion auf den Längsrichtungsversatz der zugehörigen längsgerichteten Vorrichtung in Bezug auf die optische Faser.

## Revendications

1. Système de détection de forme optique comprenant :
un fil guide (GW) comprenant une fibre optique (OF) présentant des propriétés de détection de forme optique le long d'au moins une partie de son extension longitudinale,
- un dispositif longitudinal (C) associé conçu pour permettre à un utilisateur de fixer à celui-ci le fil guide de manière à garantir que la fibre optique (OF) suivra une forme tridimensionnelle d'au moins une partie du dispositif longitudinal (C) associé,
- un système de codage du décalage longitudinal (HB) conçu pour établir un décalage longitudinal reconnaissable entre le dispositif longitudinal (C) associé et la fibre optique (OF), et
- un système de console optique (O_C, P) conçu :
- pour reconnaître ledit décalage longitudinal reconnaissable entre le dispositif longitudinal (C) associé et la fibre optique (OF), et pour enregistrer un décalage longitudinal du dispositif longitudinal (C) associé par rapport à un point le long de la fibre optique (OF) en conséquence,
- pour interroger optiquement la fibre optique (OF),
- pour reconstruire une forme tridimensionnelle d'au moins une partie de la fibre optique (OF) en réponse à un résultat de ladite interrogation optique de la fibre optique (OF), et
- pour générer une sortie (I), indicative d'une forme tridimensionnelle de l'au moins une partie du dispositif longitudinal (C) associé, en réponse à ladite forme tridimensionnelle reconstituée de l'au moins une partie de la fibre optique (OF), et en réponse audit décalage longitudinal enregistré du dispositif longitudinal (C) associé par rapport à un point le long de la fibre optique (OF).

2. Système de détection de forme optique selon la revendication 1, dans lequel le dispositif longitudinal (C) associé comprend une lumière conçue pour insérer le fil guide (GW) de manière à garantir que la fibre optique (OF) suivra une forme tridimensionnelle de l'au moins une partie (C_OF) du dispositif longitudinal (C) associé.

3. Système de détection de forme optique selon la revendication 1, conçu pour saisir au moins une propriété du dispositif longitudinal associé comprenant : une longueur et/ou une épaisseur et/ou une couleur et/ou un type, et dans lequel ledit système de détection de forme optique est conçu pour utiliser ladite au moins une propriété du dispositif longitudinal associé pour visualiser la forme tridimensionnelle du dispositif longitudinal associé à un utilisateur.

4. Système de détection de forme optique selon la revendication 1, dans lequel le fil guide (GW) présente une longueur inférieure à une longueur du dispositif longitudinal (C) associé.

5. Système de détection de forme optique selon la revendication 4, dans lequel ledit système est conçu pour déterminer une mesure de forme tridimensionnelle d'une extrémité distale du dispositif longitudinal associé en réponse à une forme reconstruite de la fibre optique (OF) et en réponse à une longueur du dispositif longitudinal (C) associé.

6. Système de détection de forme optique selon la revendication 5, dans lequel ledit système est conçu pour générer une image comportant une indication graphique de ladite mesure estimée de la forme tridimensionnelle de l'extrémité distale du dispositif longitudinal (C) associé.

7. Système de détection de forme optique selon la revendication 6, dans lequel ladite indication graphique comprend : une ligne autour d'une zone où la partie distale du dispositif longitudinal (C) associé sera très probablement présente et/ou une intensité ou un code couleur indiquant où la partie distale du dispositif longitudinal (C) associé sera très probablement présente et/ou un schéma d'une pluralité de chemins possibles que la partie distale du dispositif longitudinal (C) associé peut suivre.

8. Système de détection de forme optique selon la revendication 1, dans lequel ledit système est conçu pour estimer une mesure de la forme tridimensionnelle d'une extrémité distale du dispositif longitudinal (C) associé en réponse à un modèle d'une forme par défaut du dispositif longitudinal (C) associé.

9. Système de détection de forme optique selon la revendication 1, dans lequel le système de codage du décalage longitudinal comprend :
1) un dispositif conçu pour induire une contrainte au niveau de la fibre optique (OF) à une position connue ; et/ou 2) un système comprenant un capteur de température conçu pour être positionné à une pointe ou à un point d'entrée du dispositif longitudinal (C) associé ; et/ou 3) un système conçu pour générer une température entre deux points de la fibre optique (OF) ; et/ou 4) un système conçu pour générer une contrainte aux deux points de la fibre optique (OF) ; et/ou 5) un système comprenant un dispositif électronique.

10. Système de détection de forme optique selon la revendication 1, dans lequel le système de codage du décalage longitudinal comprend un moyeu (HB) conçu pour fournir une courbure prédéterminée de la fibre optique (OF) à une position longitudinale sélectionnée de la fibre optique (OF).

11. Système de détection de forme optique selon la revendication 10, dans lequel le système de console optique (O_C, P) est conçu pour reconnaître ladite courbure prédéterminée de la fibre optique (OF) au moyen d'une interrogation optique, et pour détecter un point le long de la fibre optique (OF) en conséquence, dans lequel ledit système de console optique (O_C, P) est en outre conçu pour enregistrer un décalage longitudinal du dispositif longitudinal (C) associé par rapport audit point détecté le long de la fibre optique (OF).

12. Système de détection de forme optique selon la revendication 10, dans lequel un moyeu (HB) est conçu pour permettre au système de console optique (O_C, P) de reconnaître une position longitudinale d'une extrémité proximale du dispositif longitudinal (C) associé sur la fibre optique (OF).

13. Système de détection de forme optique selon la revendication 1, comprenant en outre une pluralité de cathéters médicaux, lesquels peuvent être sélectionnés en tant que dispositif longitudinal (C) associé.

14. Procédé de reconstruction d'une forme d'un dispositif longitudinal associé, ledit procédé comprenant :
- la fourniture (P_GW_OF) d'un fil guide (GW) comportant une fibre optique (OF) présentant des propriétés de détection de forme optique le long d'au moins une partie de son extension longitudinale,
- la fixation (I_GW_C) du fil guide (GW) à au moins une partie d'un dispositif longitudinal (C) associé, de manière à garantir que la fibre optique (OF) suivra une forme tridimensionnelle de l'au moins une partie du dispositif longitudinal (C) associé,
- la détermination (M_HB_R) d'un décalage longitudinal reconnaissable entre le dispositif longitudinal (C) associé et la fibre optique (OF), et
- la reconnaissance (R_L_OFS) dudit décalage reconnaissable entre le dispositif longitudinal (C) associé et la fibre optique (OF), et l'enregistrement d'un décalage longitudinal du dispositif longitudinal (C) associé par rapport à un point le long de la fibre optique (OF) en conséquence,
- l'interrogation optique (O_I) de la fibre optique (OF) ,
- la reconstruction (R_3D_OF) d'une forme tridimensionnelle de l'au moins une partie de la fibre optique (OF) en réponse à un résultat de ladite interrogation optique de la fibre optique (OF), et
- la génération (G_3D_C) d'une sortie (I) indicative d'une forme tridimensionnelle de l'au moins une partie du dispositif longitudinal (C) associé en réponse à ladite forme tridimensionnelle reconstruite de l'au moins une partie de la fibre optique (OF), et en réponse audit décalage longitudinal enregistré du dispositif longitudinal (C) associé par rapport à un point le long de la fibre optique (OF).

15. Programme informatique comprenant un moyen de code pour amener un ordinateur à mettre en œuvre les étapes suivantes :
- l'enregistrement des données (M_HB_R) indiquant un décalage longitudinal entre un dispositif longitudinal associé et une fibre optique présentant des propriétés de détection de forme optique, dans lequel la fibre optique fait partie d'un fil guide,
- la réception des données (O_I) à partir d'une interrogation optique des propriétés de détection de forme optique de la fibre optique,
- la reconstruction (R_3D_OF) d'une forme tridimensionnelle de l'au moins une partie de la fibre optique en réponse auxdites données à partir de l'interrogation optique, et
- la génération d'une sortie (G_3D_C) indicative d'une forme tridimensionnelle de l'au moins une partie du dispositif longitudinal associé en réponse à ladite forme tridimensionnelle de l'au moins une partie de la fibre optique, et en réponse audit décalage longitudinal du dispositif longitudinal associé par rapport à la fibre optique.
